# DEMANDE DE BREVET EUROPEEN

(11) **EP 3 791 907 A1**
(43) Date de publication de la demande: **17.03.2021**
(21) Numéro de dépôt: 20195440.1
(22) Date de dépôt: 10.09.2020
(51) Int. Cl.: A61M 5/145, A61J 1/20, A61M 5/24

(54) **PIÈCE D'INJECTION POUR CARTOUCHE EN VERRE DE SERINGUE**

(30) Priorité: 12.09.2019 FR 1910092
(71) Demandeur: AD-HOC Scientific (Société par Actions Simplifiée à Associé Unique), 67410 Rohrwiller (FR)
(72) Inventeur: VICO, Raphaël, 67410 ROHRWILLER (FR)
(74) Mandataire: Cabinet Nuss

(57) **Abrégé**

La présente invention a pour objet une pièce d'injection pour cartouche (2) en verre operculée de seringue dont une extrémité de la cartouche (2) comprend une tête de piston (3) entourée d'un joint déplaçable dans la longueur de la cartouche (2), caractérisée en ce que la pièce d'injection est réalisée sous la forme d'une capsule monobloc (1) apte à coopérer avec le châssis d'un mécanisme d'actionnement de la tête de piston (3), de sorte que la capsule monobloc (1) présente la forme d'une cloche définissant un volume intérieur dont les dimensions intérieures sont sensiblement identiques aux dimensions extérieures de la cartouche (2) en verre, la capsule monobloc (1) comprenant :
- un orifice d'éjection (4) du contenu de la cartouche (2) positionné au sommet de la cloche qui forme la capsule monobloc (1),
- une aiguille creuse rectiligne (5) disposée à l'intérieur de la capsule monobloc (1), dont une extrémité est positionnée au niveau de l'orifice d'éjection (4) et configurée de façon à être, d'une part, insérée dans le volume intérieur de la cartouche (2) logée dans la capsule monobloc (1) et, d'autre part, alignée selon l'axe de déplacement de la tête de piston (3) de la cartouche (2).

## Description

La présente invention se rapporte au domaine des dispositifs d'injection de type seringue et plus particulièrement au domaine des dispositifs d'injection pour cartouches en verre.

Dans le cadre d'une amélioration des conditions d'hygiène en milieu hospitalier, les dispositifs d'injection à usage unique tels que les seringues se sont multipliés. Conjointement au respect de ces contraintes hygiéniques, des dispositifs d'injection ont été développés dont seuls certains éléments du dispositif présentent un caractère « jetable » parmi lesquels notamment les éléments susceptibles d'être souillés ou d'entrer en contact avec la solution à administrer. Ces dispositifs d'injection sont alors construits de façon à pouvoir détacher et remplacer les éléments « jetables » et permettre la réutilisation, voire le recyclage, des autres pièces du dispositif correspondant généralement à des pièces d'actionnement. Ainsi, des dispositifs d'injection ont été développés comprenant un réservoir sous la forme d'une cartouche amovible pré-remplie de la solution à administrer, comme dans le cas des seringues dentaires. La cartouche comprend une tête de piston à une de ses extrémités et un orifice d'éjection à l'autre extrémité qui communique avec un embout amovible de la seringue qui porte une aiguille creuse, de sorte qu'il suffit pour l'utilisateur de charger la cartouche en positionnant l'orifice d'éjection au niveau de l'embout amovible de la seringue puis d'en actionner le piston.

Toutefois, si la mise en œuvre de cette technologie fonctionne dans le cadre de dispositifs d'injection manuels, celle-ci fait apparaitre de nouvelles problématiques dans le cadre de dispositifs d'injection motorisés destinés à fonctionner de façon homogène et constante pendant plusieurs minutes, voire plusieurs heures. En effet, sous l'effet du déplacement de la tête de piston, la pression exercée par le liquide à administrer au niveau de l'orifice de la cartouche conduit à des contraintes de forces qui se trouvent démultipliées au niveau de l'embouchure de l'aiguille. Aussi, de telles contraintes de forces sont susceptibles de modifier progressivement l'axe de positionnement de l'aiguille à l'intérieur de la cartouche. Cependant, dans le cadre d'un appareillage médical, la motorisation d'un dispositif d'injection fait classiquement intervenir un actionnement avec un déplacement discontinu du piston réalisé sous la forme d'une succession de pas de déplacement identiques. En effet, lors de chaque pas, le déplacement opéré par le piston entraine une légère augmentation de la pression exercée au niveau du liquide, puis entre chaque pas, cette pression diminue par déplacement d'une partie du liquide de la cartouche au travers de l'aiguille. Il est ainsi courant en milieu médical d'opérer un actionnement du piston successivement toutes les 20 secondes selon une faible course et pendant plusieurs heures de façon à obtenir un déplacement du liquide sensiblement continu au niveau de l'aiguille. Cependant, lorsque l'axe de positionnement de l'aiguille se déplace sous l'effet de forces en contrainte, une perte de charge apparaît. Aussi, pour conserver une constance dans le débit d'éjection de liquide, la pression nécessaire à exercer doit être augmentée. Dans ces conditions, au cours d'une longue période d'injection, la corrélation entre, d'une part, le déplacement de la tête de piston et, d'autre part, le volume de liquide injecté au travers de l'aiguille est conduite à disparaitre.

La présente invention a pour but de pallier ces inconvénients en proposant un dispositif qui soit adapté à une injection continue et homogène pendant plusieurs heures ou jours d'une solution stockée dans une cartouche amovible, tout en limitant voire en supprimant les risques d'altération de la corrélation entre le déplacement de la tête de piston au niveau de la cartouche de liquide et le volume de liquide éjecté au travers de l'aiguille.

L'invention a ainsi pour objet une pièce d'injection pour cartouche en verre operculée de seringue dont une extrémité de la cartouche comprend une tête de piston entourée d'un joint déplaçable dans la longueur de la cartouche, caractérisée en ce que la pièce d'injection est réalisée sous la forme d'une capsule monobloc apte à coopérer avec le châssis d'un mécanisme d'actionnement de la tête de piston, de sorte que la capsule monobloc présente la forme d'une cloche définissant un volume intérieur dont les dimensions intérieures sont sensiblement identiques aux dimensions extérieures de la cartouche en verre, la capsule monobloc comprenant :
- un orifice d'éjection du contenu de la cartouche positionné au sommet de la cloche qui forme la capsule monobloc,
- une aiguille creuse rectiligne structurellement intégrée à la capsule monobloc et disposée à l'intérieur de la capsule monobloc, dont une extrémité est positionnée au niveau de l'orifice d'éjection et configurée de façon à être, d'une part, insérée dans le volume intérieur de la cartouche logée dans la capsule monobloc et, d'autre part, alignée selon l'axe de déplacement de la tête de piston de la cartouche.

L'invention concerne aussi un procédé de production d'une pièce d'injection selon l'invention, caractérisé en ce que le procédé comprend au moins une étape de moulage par injection de matière.

L'invention se rapporte également à un dispositif d'injection arrangé pour recevoir une cartouche en verre operculée de seringue dont une extrémité de la cartouche comprend une tête de piston entourée d'un joint déplaçable dans la longueur de la cartouche, caractérisé en ce que le dispositif comprend au moins :
- un mécanisme d'actionnement configuré pour interagir avec la tête de piston de la cartouche et translater le piston selon au moins une partie de la longueur de la cartouche, le mécanisme comprenant une pièce axiale de poussée de la tête de piston de la cartouche en direction du goulot de la cartouche,
- une pièce d'injection réalisée sous la forme d'une capsule monobloc présentant la forme d'une cloche comprenant, d'une part, un orifice d'éjection du contenu de la cartouche positionné au sommet de la cloche et, d'autre part, à l'extrémité opposée de la cloche, une interface de fixation de la pièce d'injection à un carter du mécanisme d'actionnement de façon à solidariser la capsule monobloc avec le mécanisme d'actionnement selon l'axe de translation du mécanisme d'actionnement, la capsule monobloc comprenant :
- un volume intérieur de dimensions complémentaires à celles d'une cartouche destinée à y être logée,
- une aiguille creuse rectiligne intégrée à la structure de la capsule monobloc et disposée dans son volume intérieur, de sorte qu'une extrémité est positionnée au niveau de l'orifice d'éjection, l'aiguille creuse rectiligne étant configurée de façon à être, d'une part, insérée dans le volume intérieur de la cartouche logée dans la capsule monobloc et, d'autre part, alignée selon l'axe de déplacement de la tête de piston de la cartouche.

L'invention sera mieux comprise, grâce à la description ci-après, qui se rapporte à un mode de réalisation préféré, donné à titre d'exemple non limitatif, et expliqué avec référence aux dessins schématiques annexés, dans lesquels :

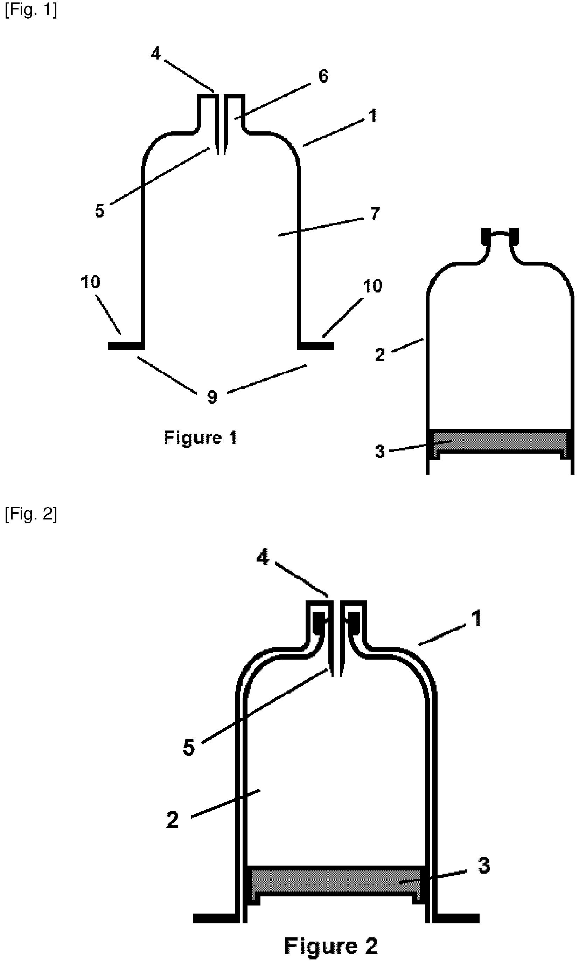

[Fig. 1] représente une vue en section d'une pièce d'injection selon l'invention destinée à être montée sur une cartouche operculée de seringue.
[Fig. 2] représente une vue en section d'une pièce d'injection selon l'invention montée sur une cartouche operculée de seringue.

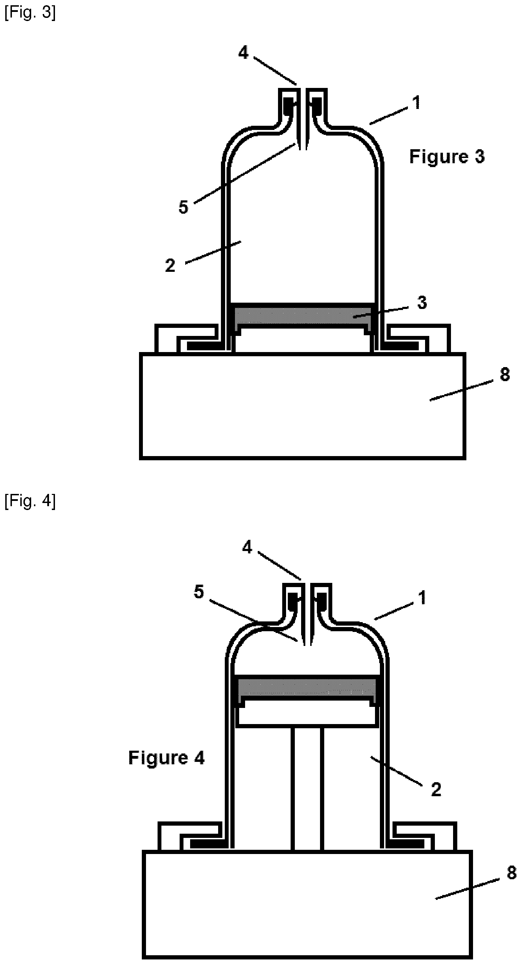

[Fig. 3] représente une vue en section d'un ensemble associant une pièce d'injection selon l'invention avec une cartouche operculée de seringue monté sur un mécanisme d'actionnement de l'injection.
[Fig. 4] représente une vue en section d'un ensemble associant une pièce d'injection selon l'invention avec une cartouche operculée de seringue monté sur un mécanisme d'actionnement de l'injection en fonctionnement.

L'invention se rapporte à une pièce d'injection pour cartouche 2 en verre operculée de seringue dont une extrémité de la cartouche 2 comprend une tête de piston 3 entourée d'un joint déplaçable dans la longueur de la cartouche 2, caractérisée en ce que la pièce d'injection est réalisée sous la forme d'une capsule monobloc 1 apte à coopérer avec le châssis d'un mécanisme d'actionnement de la tête de piston 3, de sorte que la capsule monobloc 1 présente la forme d'une cloche définissant un volume intérieur dont les dimensions intérieures sont sensiblement identiques aux dimensions extérieures de la cartouche 2 en verre, la capsule monobloc 1 comprenant :
- un orifice d'éjection 4 du contenu de la cartouche 2 positionné au sommet de la cloche qui forme la capsule monobloc 1,
- une aiguille creuse rectiligne 5 structurellement intégrée à la capsule monobloc 1 et disposée à l'intérieur de la capsule monobloc 1, dont une extrémité est positionnée au niveau de l'orifice d'éjection 4 et configurée de façon à être, d'une part, insérée dans le volume intérieur de la cartouche 2 logée dans la capsule monobloc 1 et, d'autre part, alignée selon l'axe de déplacement de la tête de piston 3 de la cartouche 2.

Grâce à ses dimensions adaptées pour entourer intimement la cartouche 2 sur laquelle elle est destinée à être montée, la capsule monobloc 1 opère un ajustement optimisé, d'une part, de la position de l'orifice d'éjection 4 par rapport à l'orifice de la cartouche 2 et, d'autre part de l'insertion de l'aiguille creuse 5 puis de son positionnement dans le volume intérieur de la cartouche 2. Lors de son actionnement, la tête de piston 3 est destinée à être déplacée le long d'un axe défini par les parois intérieures de la cartouche 2. De même, l'insertion de la cartouche 2 dans la capsule monobloc 1 de dimensions complémentaires est opérée selon un axe défini par les parois extérieures de la cartouche 2. La cartouche 2 en verre étant obtenue par soufflage, l'épaisseur de la paroi périphérique de la cartouche 2 est alors généralement homogène de sorte que l'axe d'insertion de la cartouche 2 dans la capsule monobloc 1 se trouve aligné sur l'axe de translation de la tête de piston 3. Par ailleurs, l'aiguille creuse 5 à l'intérieur de la capsule monobloc 1 est disposée selon un axe strictement parallèle aux parois internes de la capsule monobloc 1 de sorte que lors de l'insertion de l'aiguille creuse 5 dans la cartouche 2 en verre, l'aiguille 5 est insérée selon son axe d'orientation qui est aligné ou parallèle à l'axe de translation de la tête de piston 3.

La construction d'une pièce monobloc 1 qui combine, d'une part, la capsule de recouvrement pour envelopper la cartouche 2 et, d'autre part, l'aiguille creuse 5 permet d'éviter toute faiblesse au niveau de la jonction entre ces deux parties qui serait susceptible, d'une part, d'entraîner une modification de l'axe de positionnement de l'aiguille 5 insérée ou en cours d'insertion dans la cartouche 2 et, d'autre part, l'apparition d'une fuite au niveau de la jonction entre ces deux parties. Aussi, la réalisation d'une structure monobloc 1 permet d'assurer un maintien de la position de l'aiguille creuse 5 et notamment de l'orientation de l'axe de cette aiguille 5 par rapport au reste de la capsule 1 destinée à envelopper la cartouche 2. Lorsque la cartouche 2 est insérée dans la capsule monobloc 1 jusqu'à ce que le goulot de la cartouche 2 entre en butée au niveau de l'orifice d'éjection 4 de la capsule 1, le dimensionnement complémentaire du volume intérieur de la capsule 1 par rapport au volume extérieur de la cartouche 2 permet un assemblage selon lequel la capsule 1 et la cartouche 2 sont solidaires à toute translation dans un plan perpendiculaire à l'axe de l'assemblage de la cartouche 2 avec la capsule monobloc 1. Selon une spécificité de ce dimensionnement complémentaire de la cartouche 2 avec la capsule monobloc 1, la cartouche 2 enchâssée dans la capsule monobloc 1 s'y trouve bloquée par friction de sorte qu'une fois le contenu de la cartouche 2 éjecté, la capsule monobloc 1 est éliminée avec la cartouche 2 vide. La capsule monobloc 1 présente ainsi un fonctionnement à usage unique. Par ailleurs, conjointement à cet assemblage de la cartouche 2 avec la capsule monobloc 1, grâce à la complémentarité dimensionnelle, l'aiguille creuse 5 positionnée au niveau de l'orifice d'éjection 4 est introduite dans le volume intérieur de la cartouche 2 par son goulot. L'axe de l'aiguille creuse 5 est ainsi stabilisé par rapport à la cartouche 2 grâce à l'ensemble réalisé par la capsule monobloc 1 qui enchâsse la cartouche 2. L'aiguille creuse 5 demeure ainsi inamovible tout en supportant une pression sensiblement continue pendant des durées de plusieurs heures, cette pression étant exercée notamment par le contenu de la cartouche 2 éjecté sous l'action de la tête de piston 3 en déplacement. L'inamovibilité de l'aiguille 5 par rapport à la capsule 1 évite ainsi à l'aiguille une déviation de son axe orienté de façon parallèle à l'axe de translation de la tête de piston 3. Aussi, en absence d'inclinaison de l'axe de l'aiguille 5 insérée, le risque de perte de charge dans le flux de liquide en éjection continue au niveau de l'aiguille creuse 5 se trouve réduit voire supprimé, de sorte que la corrélation entre, d'une part, le déplacement de la tête de piston 3 et, d'autre part, le volume de liquide en déplacement au travers de l'aiguille 5 se trouve conservée pendant une durée d'éjection de liquide de plusieurs heures.

Selon un exemple préféré de mise en œuvre, la capsule monobloc 1 présente une symétrie axiale centrée sur l'axe d'insertion d'une cartouche 2 dans cette capsule 1. L'orifice d'éjection 4 de la capsule 1 se trouve alors centré sur l'axe de symétrie de la capsule monobloc 1, selon une complémentarité de position du goulot par rapport au corps de la cartouche 2, de sorte que l'insertion de la cartouche 2 dans la capsule monobloc 1 aligne conjointement l'orifice d'éjection 4 et donc l'aiguille creuse 5 avec le goulot de la cartouche 2.

Selon un exemple particulier de réalisation de la pièce d'injection de l'invention, le volume intérieur de la capsule 1 comprend au moins, d'une part, un premier compartiment 6 de plus petite section au niveau duquel est positionné l'orifice d'éjection 4 et dont les dimensions sont complémentaires du goulot et/ou du col de la cartouche 2 et, d'autre part, un second compartiment 7 de plus grande section dont les dimensions sont complémentaires de l'épaule et du corps de la cartouche 2. Une telle construction optimise le positionnement de la capsule monobloc 1 au niveau du goulot et/ou du col de la cartouche 2 grâce à un montage intime de la portion de la capsule 1 qui porte l'orifice d'éjection 4 et l'aiguille creuse 5 de sorte que la stabilisation, voire la solidarisation, de l'aiguille creuse 5 par rapport à la cartouche 2 notamment au niveau de l'orifice d'éjection 4 se trouve optimisée. Cet assemblage optimal de la capsule monobloc 1 avec la cartouche 2 permet de supprimer ainsi tout risque de déplacement, notamment par rapport à l'axe d'insertion de l'aiguille creuse 5 ou par rapport à l'axe de déplacement de la tête de piston 3.

Selon un autre exemple particulier de réalisation de la pièce d'injection de l'invention, compatible avec l'exemple particulier précédent, l'aiguille intérieure creuse 5 comprend une longueur qui est, d'une part, suffisamment importante pour traverser l'opercule de la cartouche 2 et, d'autre part, suffisamment courte pour ne pas empêcher un déplacement de la tête de piston 3 de la cartouche 2 jusqu'à une épaule de la cartouche 2. Selon cet exemple de réalisation, la longueur de l'aiguille creuse 5 correspond, au plus, à la hauteur qui sépare l'orifice de la cartouche 2 du rétrécissement de la section intérieure de la cartouche 2 en lien avec la présence de l'épaule de cette cartouche 2. Une telle construction permet ainsi à la tête de piston 3 d'être translatée le long d'une course maximale dans la longueur de la cartouche 2 sans être limitée par un contact avec l'extrémité de l'aiguille creuse 5 insérée dans la cartouche 2 depuis le goulot de cette cartouche 2. Cette course optimisée du déplacement de la tête de piston 3 dans la longueur de la cartouche 2 autorise ainsi la translation de la tête de piston 3 jusqu'à être stoppée par une réduction de la section intérieure de la cartouche 2, telle que l'épaule de la cartouche 2, de sorte que le résidu du contenu de la cartouche 2 qui n'est pas éjecté est réduit à son minimum.

Selon un autre exemple particulier de réalisation de la pièce d'injection de l'invention, également compatible avec les exemples particuliers précédents, l'extrémité de l'aiguille creuse 5 opposée à l'orifice d'éjection présente une section en biseau. Cette section en biseau permet à la capsule monobloc 1 de faciliter le percement de l'opercule au niveau du goulot de la cartouche 2 lorsque la cartouche 2 est insérée dans la capsule monobloc 1.

Selon un autre exemple particulier de réalisation de la pièce d'injection de l'invention, également compatible avec les exemples particuliers précédents, l'orifice d'éjection 4 positionné sur la face extérieure de la capsule monobloc 1 présente une interface de connexion de type Luer lock universelle. Ce type d'interface, également normée ISO 594-1 et 2, permet d'opérer la connexion d'une interface complémentaire par simple mouvement de vrille d'une structure femelle sur une structure mâle, tout en assurant une étanchéité lors de transfert du contenu de la cartouche 2 au travers de l'aiguille intérieure creuse 5, en sortie de l'orifice d'éjection 4.

Selon un autre exemple particulier de réalisation de la pièce d'injection de l'invention, complémentaire des exemples particuliers précédents détaillés, la capsule monobloc 1 est réalisée à partir de plastique injecté. De façon préférentielle, le plastique injecté est un polymère thermoplastique par exemple de type acrylonitrile butadiène styrène ou ABS, idéalement un ABS de grade médical également référencé sous la dénomination M-ABS.

L'invention se rapporte également un procédé de production d'une capsule monobloc 1 selon l'invention. Ce procédé est caractérisé en ce qu'il comprend au moins une étape de moulage par injection de matière. Un tel procédé présente comme avantage de permettre une production à grande échelle de capsules monoblocs 1 intégrant une aiguille creuse 5 selon un dimensionnement reproductible et parfaitement adapté à une cartouche 2 en verre operculée dont le format est défini.

L'invention concerne aussi un dispositif d'injection arrangé pour recevoir une cartouche 2 en verre operculée de seringue dont une extrémité de la cartouche 2 comprend une tête de piston 3 entourée d'un joint déplaçable dans la longueur de la cartouche 2, caractérisé en ce que le dispositif comprend au moins :
- un mécanisme d'actionnement 8 configuré pour interagir avec la tête de piston 3 de la cartouche 2 et translater le piston 3 selon au moins une partie de la longueur de la cartouche 2, le mécanisme 8 comprenant une pièce axiale de poussée de la tête de piston 3 de la cartouche 2 en direction du goulot de la cartouche 2,
- une pièce d'injection réalisée sous la forme d'une capsule monobloc 1 présentant la forme d'une cloche comprenant, d'une part, un orifice d'éjection 4 du contenu de la cartouche 2 positionné au sommet de la cloche et, d'autre part, à l'extrémité opposée de la cloche, une interface de fixation 9 de la pièce d'injection à un carter du mécanisme d'actionnement 8 de façon à solidariser la capsule monobloc 1 avec le mécanisme d'actionnement 8 selon l'axe de translation du mécanisme d'actionnement 8, la capsule monobloc 1 comprenant :
   - un volume intérieur de dimensions complémentaires à celles d'une cartouche destinée à y être logée,
   - une aiguille creuse rectiligne 5 intégrée à la structure de la capsule monobloc 1 et disposée dans son volume intérieur, de sorte qu'une extrémité est positionnée au niveau de l'orifice d'éjection 4, l'aiguille creuse rectiligne 5 étant configurée de façon à être, d'une part, insérée dans le volume intérieur de la cartouche 2 logée dans la capsule monobloc 1 et, d'autre part, alignée selon l'axe de déplacement de la tête de piston 3 de la cartouche 2.

Le dispositif d'injection fait coopérer la capsule monobloc 1 de l'invention avec un mécanisme d'actionnement 8 pour opérer l'éjection de contenu d'une cartouche 2 enchâssée dans une capsule 1. Cette coopération se réalise par une solidarisation de la capsule monobloc 1 avec le mécanisme d'actionnement 8 de sorte que tout déplacement parasite de la capsule monobloc 1 par rapport au châssis du mécanisme d'actionnement 8 soit impossible afin d'éviter une déviation de la translation opérée par le mécanisme d'actionnement 8 par rapport à la capsule monobloc 1 et à l'axe de l'aiguille creuse rectiligne 5. De plus, la complémentarité dimensionnelle du volume intérieur de la capsule monobloc 1 et de la structure en verre de la cartouche 2 réalise un logement dans lequel la cartouche 2 se trouve insérée et bloquée en position dans la capsule monobloc 1. Cette immobilisation de la cartouche 2 dans le logement de la capsule monobloc 1 empêche tout débattement de la cartouche 2 par rapport à la capsule monobloc 1 et au mécanisme d'actionnement 8 et donc par rapport à l'aiguille 5 de la capsule 1. Aussi, l'insertion de la cartouche 2 à l'intérieur de la capsule 1 assure conjointement un verrouillage de la cartouche 2 dans le dispositif d'injection selon la position nécessaire et optimale pour l'éjection de son contenu et empêche toute erreur de positionnement de la cartouche 2, voire même toute déviation de la course de la tête de piston 3 par rapport à l'axe de l'aiguille creuse rectiligne 5 de la capsule monobloc 1 au cours de sa translation axiale entraînée par le mécanisme d'actionnement 8.

Selon un exemple particulier de construction du dispositif d'injection, le mécanisme d'actionnement 8 comprend également un moyen moteur configuré pour opérer un déplacement homogène de la tête de piston 3 de la cartouche 2 de sorte que la course de la tête de piston 3 à l'intérieur de la cartouche 2 est parcourue au cours d'une durée supérieure à plusieurs minutes, voire plusieurs heures. Le dispositif d'injection selon l'invention est ainsi configuré pour opérer une injection continue du contenu de la cartouche 2 par déplacement de la tête de piston 3 avec une vitesse constante de façon à obtenir un débit d'éjection du contenu de la cartouche 2 qui, bien que susceptible d'être de faible importance, soit constant pendant des intervalles de temps correspondant à plusieurs minutes, voire plusieurs heures.

Il convient de relever que l'intégration structurelle de l'aiguille creuse 5 rectiligne dans la capsule monobloc 1 en combinaison avec la complémentarité de forme de la cartouche 2 avec la capsule monobloc 1 solidarisée au mécanisme d'actionnement 8 permet d'opérer un placement et un maintien en position optimisée de la cartouche 2 et l'axe de déplacement de la tête de piston 3 par rapport à l'axe de l'aiguille creuse 5 de la capsule monobloc 1. Ce maintien de la position de l'aiguille 5 par rapport à l'axe de la course de la tête de piston 3 dans la cartouche 2 permet d'assurer une pression uniforme et constante au niveau de l'ensemble de l'aiguille 5 lors de l'opération d'éjection du contenu de la cartouche 2 sous l'effet du déplacement de la tête de piston 3. A l'inverse d'un dispositif d'injection configuré pour une injection instantanée avec un déplacement en quelques secondes de la tête de piston 3 le long de sa course, l'aiguille creuse 5 à l'intérieur de la capsule monobloc 1 doit être en mesure de supporter pendant une durée de plusieurs minutes, voire plusieurs heures, une pression constante susceptible de la déformer et/ou de la désaxer. L'intégration de l'aiguille creuse 5 dans la capsule 1 sous la forme d'une structure monobloc permet la réalisation d'une aiguille 5 construite sans point de faiblesse, c'est-à-dire adaptée pour supporter une pression continue pendant plusieurs minutes, voire plusieurs heures, et dont le positionnement dans la cartouche 2 est assuré d'être selon une disposition optimisée lorsque la cartouche 2 est insérée à l'intérieur de la capsule monobloc 1 et que la capsule monobloc 1 est fixée par son interface 9 dédiée sur le carter du mécanisme d'actionnement 8.

L'extrémité de la pièce d'injection réalisée par la capsule monobloc 1 participe par l'interface de fixation 9 à la solidarisation de la capsule monobloc 1 avec le mécanisme d'actionnement 8. Cette solidarisation assure un verrouillage de la capsule monobloc 1 avec un carter du mécanisme d'actionnement 8 selon un axe de translation correspondant sensiblement à l'axe de déplacement de la tête de piston 3 dans la cartouche 2. Aussi, le mécanisme d'actionnement 8 exerce une poussée continue au niveau de la tête de piston 3 tandis que la cartouche 2 en verre est bloquée en translation à l'intérieur de la capsule monobloc 1, elle-même fixée au carter du mécanisme d'actionnement 8. La poussée exercée au niveau de la tête de piston 3 entraine ainsi le coulissement de la tête de piston 3 à l'intérieur du corps de la cartouche 2 et l'expulsion du contenu de cette cartouche 2 au travers de l'aiguille creuse 5 et de l'orifice d'éjection 7 de la capsule monobloc 1.

Selon un premier exemple particulier de réalisation de l'interface de fixation 9, celle-ci est réalisée sous la forme d'une surface filetée ou taraudée qui interagit avec une surface taraudée ou filetée portée par le carter du mécanisme d'actionnement 8. Selon un second exemple particulier, alternatif à l'exemple particulier de réalisation précédent, l'interface de fixation 9 est réalisée par une ou plusieurs ailettes 10 disposées dans un plan sensiblement perpendiculaire à l'axe de l'aiguille creuse 5 à l'intérieur de la capsule monobloc 1. Ces ailettes 10 sont arrangées pour coopérer avec une interface de type baïonnette portée par le carter du mécanisme d'actionnement 8.

Selon un exemple particulier de construction, le mécanisme d'actionnement 8 intègre une pièce axiale montée mobile en translation sous l'action d'un moyen moteur et dont une extrémité est arrangée pour interagir avec la face extérieure de la tête du piston 3, c'est-à-dire une surface de la tête du piston 3 qui n'est ni en contact avec le contenu de la cartouche 2, ni avec une des parois de la cartouche 2.

Selon un exemple particulier de construction du dispositif d'injection de l'invention, compatible avec les exemples particuliers précédents, la pièce axiale du mécanisme d'actionnement 8 de la translation de la tête de piston 3 de la cartouche 2 comprend une surface de contact configurée pour coopérer avec une surface de la tête de piston 3 opposée au volume intérieur de la cartouche 2, la surface de contact du mécanisme d'actionnement présentant un relief sensiblement complémentaire du relief de la surface de la tête de piston 3. La translation de la tête de piston 3 à l'intérieur de la cartouche 2 étant effectuée de façon unidirectionnelle, l'interaction de l'extrémité de la pièce axiale du mécanisme d'actionnement ne fonctionne alors que dans le cadre d'une poussée de l'extrémité de la pièce axiale contre la tête de piston 3. Aussi, aucune fixation particulière de la pièce axiale à la tête de piston 3 n'est recherchée de sorte qu'en fin de course, la translation inverse de la pièce axiale entraîne la séparation de son extrémité d'avec la tête de piston 3 qui reste en position à l'intérieur de la cartouche 2. De plus, lorsque la capsule monobloc 1 qui loge la cartouche 2 est détachée du carter du mécanisme d'actionnement 8, la cartouche 2 est également séparée sans difficulté de la pièce axiale de ce mécanisme 8.

Selon un autre exemple particulier de construction du dispositif d'injection de l'invention, la surface de contact du mécanisme d'actionnement 8 de la translation est sensiblement identique à celle de la surface de la tête de piston 3 opposée au volume intérieur de la cartouche 2. Cette identité de surface permet notamment au mécanisme d'actionnement d'exercer une pression sur l'ensemble de la section de la tête du piston 3. Cette homogénéité de pression sur l'ensemble de la section de la tête du piston participe à la réalisation d'un déplacement homogène en translation de la tête du piston 3 à l'intérieur de la cartouche 2, d'une part, de manière à générer une pression également répartie à l'intérieur de la cartouche 2 et donc exercée au niveau de l'aiguille creuse 5 et, d'autre part, de manière à éliminer le contenu de la cartouche 2 d'une façon optimale en limitant tout résidu du contenu au niveau de la jonction de la tête du piston 3 avec les parois internes de la cartouche 2.

Le dispositif d'injection de l'invention, dans ses différentes variantes et exemples de construction, est adapté pour fonctionner avec une pièce d'injection pour cartouche 2 de la forme d'une capsule monobloc 1 selon l'invention.

Bien entendu, l'invention n'est pas limitée au mode de réalisation décrit et représenté aux dessins annexés. Des modifications restent possibles, notamment du point de vue de la constitution des divers éléments ou par substitution d'équivalents techniques, sans sortir pour autant du domaine de protection de l'invention.

## Revendications

1. Pièce d'injection pour cartouche (2) en verre operculée de seringue dont une extrémité de la cartouche (2) comprend une tête de piston (3) entourée d'un joint déplaçable dans la longueur de la cartouche (2), **caractérisée en ce que** la pièce d'injection est réalisée sous la forme d'une capsule monobloc (1) apte à coopérer avec le châssis d'un mécanisme d'actionnement de la tête de piston (3), de sorte que la capsule monobloc (1) présente la forme d'une cloche définissant un volume intérieur dont les dimensions intérieures sont sensiblement identiques aux dimensions extérieures de la cartouche (2) en verre, la capsule monobloc (1) comprenant :
- un orifice d'éjection (4) du contenu de la cartouche (2) positionné au sommet de la cloche qui forme la capsule monobloc (1),
- une aiguille creuse rectiligne (5) structurellement intégrée à la capsule monobloc (1) et disposée à l'intérieur de la capsule monobloc (1), dont une extrémité est positionnée au niveau de l'orifice d'éjection (4) et configurée de façon à être, d'une part, insérée dans le volume intérieur de la cartouche (2) logée dans la capsule monobloc (1) et, d'autre part, alignée selon l'axe de déplacement de la tête de piston (3) de la cartouche (2).

2. Pièce d'injection selon la revendication 1, **caractérisée en ce que** le volume intérieur de la capsule (1) comprend au moins, d'une part, un premier compartiment (6) de plus petite section au niveau duquel est positionné l'orifice d'éjection (4) et dont les dimensions sont complémentaires du goulot et/ou du col de la cartouche (2) et, d'autre part, un second compartiment (7) de plus grande section dont les dimensions sont complémentaires de l'épaule et du corps de la cartouche (2).

3. Pièce d'injection selon une des revendications précédentes, **caractérisée en ce que** l'aiguille intérieure creuse (5) comprend une longueur qui est, d'une part, suffisamment importante pour traverser l'opercule de la cartouche (2) et, d'autre part, suffisamment courte pour ne pas empêcher un déplacement de la tête de piston (3) de la cartouche (2) jusqu'à une épaule de la cartouche (2).

4. Pièce d'injection selon une des revendications précédentes, **caractérisée en ce que** l'extrémité de l'aiguille creuse (5) opposée à l'orifice d'éjection présente une section en biseau.

5. Pièce d'injection selon une des revendications précédentes, **caractérisée en ce que** l'orifice d'éjection (4) positionné sur la face extérieure de la capsule monobloc (1) présente une interface de connexion de type Luer lock universelle.

6. Pièce d'injection selon une des revendications précédentes, **caractérisée en ce que** la capsule monobloc (1) est réalisée à partir de plastique injecté.

7. Procédé de production d'une pièce d'injection selon une des revendications 1 à 6, **caractérisé en ce que** le procédé comprend au moins une étape de moulage par injection de matière.

8. Dispositif d'injection arrangé pour recevoir une cartouche (2) en verre operculée de seringue dont une extrémité de la cartouche (2) comprend une tête de piston (3) entourée d'un joint déplaçable dans la longueur de la cartouche (2), **caractérisé en ce que** le dispositif comprend au moins :
- un mécanisme d'actionnement (8) configuré pour interagir avec la tête de piston (3) de la cartouche (2) et translater le piston (3) selon au moins une partie de la longueur de la cartouche (2), le mécanisme (8) comprenant une pièce axiale de poussée de la tête de piston (3) de la cartouche (2) en direction du goulot de la cartouche (2),
- une pièce d'injection réalisée sous la forme d'une capsule monobloc (1) présentant la forme d'une cloche comprenant, d'une part, un orifice d'éjection (4) du contenu de la cartouche (2) positionné au sommet de la cloche et, d'autre part, à l'extrémité opposée de la cloche, une interface de fixation (9) de la pièce d'injection à un carter du mécanisme d'actionnement (8) de façon à solidariser la capsule monobloc (1) avec le mécanisme d'actionnement (8) selon l'axe de translation du mécanisme d'actionnement (8), la capsule monobloc (1) comprenant :
- un volume intérieur de dimensions complémentaires à celles d'une cartouche destinée à y être logée,
- une aiguille creuse rectiligne (5) intégrée à la structure de la capsule monobloc (1) et disposée dans son volume intérieur, de sorte qu'une extrémité est positionnée au niveau de l'orifice d'éjection (4), l'aiguille creuse rectiligne (5) étant configurée de façon à être, d'une part, insérée dans le volume intérieur de la cartouche (2) logée dans la capsule monobloc (1) et, d'autre part, alignée selon l'axe de déplacement de la tête de piston (3) de la cartouche (2).

9. Dispositif d'injection selon la revendication 8, **caractérisé en ce que** le mécanisme d'actionnement (8) comprend également un moyen moteur configuré pour opérer un déplacement homogène de la tête de piston (3) de la cartouche (2) de sorte que la course de la tête de piston (3) à l'intérieur de la cartouche (2) est parcourue au cours d'une durée supérieure à plusieurs minutes, voire plusieurs heures.

10. Dispositif d'injection selon une des revendications précédentes, **caractérisé en ce que** la pièce axiale du mécanisme d'actionnement (8) de la translation de la tête de piston (3) de la cartouche (2) comprend une surface de contact configurée pour coopérer avec une surface de la tête de piston (3) opposée au volume intérieur de la cartouche (2), la surface de contact du mécanisme d'actionnement présentant un relief sensiblement complémentaire du relief de la surface de la tête de piston (3).

11. Dispositif d'injection selon la revendication 10, **caractérisé en ce que** la surface de contact du mécanisme d'actionnement (8) de la translation est sensiblement identique à celle de la surface de la tête de piston (3) opposée au volume intérieur de la cartouche (2).
